# EUROPÄISCHE PATENTSCHRIFT

(11) **EP 2 708 240 B1**
(45) Veröffentlichungstag und Bekanntmachung des Hinweises auf die Patenterteilung: **26.04.2017**
(21) Anmeldenummer: 13184111.6
(22) Anmeldetag: 12.09.2013
(51) Int. Cl.: A61K 39/108, A23K 20/195, A23K 20/147, A23K 50/30

(54) **Behandlung von Schwanznekrosen**
Treatment of tail necrosis
Traitement de nécroses de la queue

(30) Priorität: 12.09.2012 DE 102012017983; 29.12.2012 EP 12199789
(43) Veröffentlichungstag der Anmeldung: 19.03.2014
(73) Patentinhaber: Jaeger, Friedhelm, 40670 Meerbusch (DE)
(72) Erfinder: Jaeger, Friedhelm, 40670 Meerbusch (DE)
(74) Vertreter: Roth, Andy Stefan

(56) Entgegenhaltungen:
- WO-A1-00/75345
- US-A1- 2008 085 287
- G. Z. PANOS ET AL: "Systematic review: are antibiotics detrimental or beneficial for the treatment of patients with Escherichia coli O157:H7 infection?", ALIMENTARY PHARMACOLOGY & THERAPEUTICS, Bd. 24, Nr. 5, 1. September 2006 (2006-09-01), Seiten 731-742, XP55089669, ISSN: 0269-2813, DOI: 10.1111/j.1365-2036.2006.03036.x
- KIYOTAKA NISHIKAWA: "Recent Progress of Shiga Toxin Neutralizer for Treatment of Infections by Shiga Toxin-Producing", ARCHIVUM IMMUNOLOGIAE ET THERAPIAE EXPERIMENTALIS, BIRKHAEUSER-VERLAG, BASEL, CH, Bd. 59, Nr. 4, 5. Juni 2011 (2011-06-05), Seiten 239-247, XP019917229, ISSN: 1661-4917, DOI: 10.1007/S00005-011-0130-5
- SIU-KEI CHOW ET AL: "Monoclonal Antibodies and Toxins-A Perspective on Function and Isotype", TOXINS, Bd. 4, Nr. 12, 11. Juni 2012 (2012-06-11), Seiten 430-454, XP055090041, ISSN: 2072-6651, DOI: 10.3390/toxins4060430
- CROSS A S ET AL: "Development of an anti-core lipopolysaccharide vaccine for the prevention and treatment of sepsis", VACCINE, ELSEVIER LTD, GB, Bd. 22, Nr. 7, 17. Februar 2004 (2004-02-17), Seiten 812-817, XP004487433, ISSN: 0264-410X, DOI: 10.1016/J.VACCINE.2003.11.025
- M. F. SANTOS ET AL: "Lipopolysaccharide as an Antigen Target for the Formulation of a Universal Vaccine against Escherichia coli O111 Strains", CLINICAL AND VACCINE IMMUNOLOGY, Bd. 17, Nr. 11, 1. November 2010 (2010-11-01), Seiten 1772-1780, XP55089972, ISSN: 1556-6811, DOI: 10.1128/CVI.00232-10
- "Vorstellung des laufenden "Kleeschwein"-Praxisversuchs: Umsetzung von Praxisversuchen zur Untersuchung der Verfütterung von Kleesilage und Luzernegrünmehl in der Bioschweinefütterung (Projektlaufzeit 07/2008- 12/2010)", , 1. Januar 2010 (2010-01-01), XP55090014, Gefunden im Internet: URL:http://www.fibl.org/fileadmin/document s/de/oesterreich/arbeitsschwerpunkte/Tierh altung/Kleeschwein_TB_BA-Bauerntage2010.pd f [gefunden am 2013-11-25]
- PHYLLIS P SUN ET AL: "Endotoxin-binding affinity of sevelamer: a potential novel anti-inflammatory mechanism", KIDNEY INTERNATIONAL, Bd. 76, 1. Dezember 2009 (2009-12-01), Seiten S20-S25, XP55089974, ISSN: 0085-2538, DOI: 10.1038/ki.2009.403
- FRIEDHELM JAEGER: "Das Projekt "intakter Ringelschwanz" beim Schwein - stehen wir vor dem Durchbruch?", TIERÄRZTL. UMSCHAU, Bd. 68, 1. Januar 2013 (2013-01-01), Seiten 3-11, XP055089457,

## Beschreibung

Die vorliegende Erfindung betrifft Substanzen zur therapeutischen oder/und prophylaktischen Behandlung von Schwanznekrosen bei Tieren, insbesondere von Schwanznekrosen bei Schweinen, sowie Verfahren zur therapeutischen und/oder prophylaktischen Behandlung von Tieren, die von Schwanznekrosen betroffen sind oder bei denen die Gefahr einer Schwanznekrose besteht.

### Hintergrund

Schwanznekrosen kommen bei einer Vielzahl von Tieren vor und sind durch ringförmige Einschnürungen und Absterben (Nekrose) der betroffenen, distalen Schwanzabschnitte gekennzeichnet. Bekannt sind Schwanznekrosen in der Masttierhaltung insbesondere bei Rindern und Schweinen.

Die Schwanznekrose bei Schweinen ist dabei eine sehr häufig vorkommende eitrig-nekrotisierende Entzündung der Schwanzspitze. Im Laufe der ersten Lebenswochen bildet sich am caudalen Ende des Schwanzes betroffener Ferkel eine ringförmige, rötlich-verfärbte Auftreibung, worauf der distal gelegene Schwanzabschnitt einer trockenen Nekrose verfällt, die sich bei sekundärer bakterieller Besiedlung jedoch auch entzündlich-exsudativ entwickeln kann. Die Nekrose ist deutlich demarkiert vom gesunden Schwanzgewebe abgesetzt und der nekrotische caudale Teil des Schwanzes fällt normalerweise in den weiteren Lebenswochen ab oder wird von den Buchtengenossen abgefressen.

Problem an der Schwanznekrose bei Ferkel ist, dass Ferkel bei denen die Schwänze entzündet, zunächst ein auffälliges Verhalten zeigen, beispielsweise ein Einklemmen des wohl schmerzhaften Schwanzes und deutliche Symptome von Juckreiz. Man konnte feststellen, dass dieses auffällige Verhalten andere Buchtengenossen insbesondere im Aufzucht- und im Mastbereich auf die nekrotischen Schweineschwänze aufmerksam werden lässt.

Der Juckreiz führt dabei zum Dulden eines neugierigen "Beknabberns" durch den Buchtengenossen, was zu einer Verstärkung des Entzündungsprozesses führt. Durch den Austritt von Gewebeflüssigkeit aus dem entzündeten, absterbenden Gewebe bis hin zu Blutungen kommt es so zu einem *circulus vitiosus,* in dessen Verlauf in die entzündliche Stelle auch sekundär Umweltkeime eingetragen werden. Dies kann zur Unterentwicklung der betroffenen Tiere führen infolge innerer Entzündungen und Abszessbildung als aufsteigende Infektion vom infizierten Schwanzstummel ausgehend, was unter anderem auch zu erhebliche Schmerzen bei den Tieren bis hin zum Tode führt.

Erreichen die betroffenen Tiere gleichwohl die Schlachtreife, kann es ferner zum Verlust wichtiger Teilstücke oder gar des ganzen Schlachttierkörpers ergeben. Dies betrifft 0,5% - 3,4% der Schlachtschweine; bei insgesamt 60 Mio. Schlachtungen/Jahr allein in Deutschland sind dies hierzulande mehr als 300.000 Tierkörper/Jahr. Hinzu kommen die Verluste in den schweinehaltenden Betrieben aufgrund Minderleistung, tierärztlichen Behandlungen und Tierverlusten. Bezogen auf das gesamte System der Schweineerzeugung und Vermarktung werden die Gesamtkosten auf € 9,26 pro Schwein geschätzt, wobei allein im Mastbereich die ökonomischen Verluste auf einzelbetrieblicher Ebene bei € 2,425 pro Mastschwein liegen (Präsentation der Studie *EUWE*/*Net,* vorgestellt auf einen EU-Workshop am 9. September 2013).

Die genaue Ursache, die zu Schwanznekrosen bei ungekürzten Ferkelschwänzen führt, ist bisher nicht bekannt. Am Entstehen von zu der Schwanznekrose verwandten Krankheiten wie "Nekrosen der Schwanzstummel" und "Ohrrandnekrosen" (beides auch als Nekrosen der "Akren" bezeichnet), die sowohl beim Mastschwein als auch beim Ferkel vorkommen, sind nach gängiger Lehrmeinung zahlreiche Faktoren beteiligt; es handelt sich demnach um ein multifaktorielles Geschehen. Als Auslöser werden zumeist infektiöse Ursachen (z.B. PRRSV oder Circoviren, Mykoplasmen), Mykotoxine, Toxine anderer Herkunft sowie vor allem auch Umwelteinflüsse (insbesondere Lüftungsfehler) einschließlich Haltungs-, Management- und Fütterungsfehler, die in Verbindung mit anderen Grundkrankheiten dann zum Entstehen von Nekrosen führen können (Tiermedizinische Mikrobiologie, Infektions- und Seuchenlehre, Hans-Joachim Selbitz, Uwe Truyen, Peter Valetin-Weigand, 9. Auflage, S.187 ff, Verlag Enke). Alle diese Theorien haben zweifellos ihre Berechtigung, geben aber keine Auskunft darüber, welche der jeweiligen Einflussfaktoren letztlich der ausschlaggebende ist Und vor allem: ob die Erkenntnisse zur "Ohrrand-Nekrose" beim Ferkel überhaupt auf krankhafte Prozesse im ungekürzten Ferkelschwanz übertragen werden können und inwieweit letztere überhaupt in relevanter Häufigkeit auftreten, ist bisher nicht bekannt.

Sämtliche bisherigen Versuche, die Schwanznekrose mit Vitaminen, Spurenelementen, Antibiotika und Chemotherapeutika zu verhindern, brachten keinen Erfolg. Die zurzeit einzige gängige "Prophylaxe" ist das vorsorgliche Kürzen des Schwanzes beim Ferkel durch Amputation am 4. Lebenstag, was jedoch als "Routinemaßnahme" EU-rechtlich verboten ist (Jaeger, F.: Der Tierarzt als berufener Tierschützer - Wie wird man diesem Anspruch wirklich gerecht?, Deutsches Tierärzteblatt, 7/2011).

Nicht zuletzt auch aufgrund eines Beschwerdeverfahrens der Europäischen Kommission gegenüber einzelnen Mitgliedstaaten (einschl. Deutschland) und diverser Eingaben und Klagen von Tierschützern ist man derzeit angestrengt bemüht, das Problem des Schwanzbeißens zu lösen (Jaeger, F. Zootechnische Maßnahmen bei Nutztieren - wirklich ein MUSS? - 30. AFT-Tagung "Aktuelle Probleme des Tierschutzes", 16./17.9.2010, Seite 13).

Unter den derzeit üblichen Rahmenbedingungen muss in konventionellen Tierhaltungen vermutlich bei bis zu etwa 60% der Ferkel auf dem Flatdeck mit Schwanznekrosen / Schwanzbeißen gerechnet werden, würde man die Schwänze nicht wie derzeit am 4. Lebenstag "prophylaktisch" kürzen sondern diese lang belassen.

Die Auswirkungen dieser Erkenntnis sind gravierend und würden - falls nicht ursächlich gelöst - letztlich alle Bemühungen der Mastbetriebe, auf das Kürzen von Schweineschwänzen zu verzichten, ins Leere gehen lassen. Denn selbst wenn es in den Mastbetrieben gelingt, Maßnahmen zu entwickeln, die ein Mästen auch von "langschwänzigen" Schweinen erlauben, könnte dies dennoch nicht zum Erfolg führen, wenn es nicht zugleich auch gelingt, entsprechend "unbehandelte" Ferkel zu erzeugen. Wenn aber bei geschätzt etwa 60% der Aufzuchtferkel die Schwänze entzündlich/nekrotisch werden (gezielte Untersuchungen lassen sogar eine noch höhere Prävalenz erwarten), wird es gar nicht möglich sein, den Mastbetrieben in ausreichender Zahl unkupierte Ferkel zu liefern.

Unabhängig davon haben Nekrosen von Schwänzen auch bei Aufzuchtferkeln unmittelbar erhebliche ökonomische Auswirkungen, weil kranke Tiere in ihrer Leistung zurückbleiben; sie werden zumeist als Kümmerer gemerzt bzw. bleiben später in der Mast zurück.

Nekrotisierende Schwänze sind zudem ein wichtiger Indikator dafür, ob sich die Tiere wohl fühlen oder ob sie "krank" sind. Die Schwänze stellen somit ein wichtiges tiergesundheitliches "Frühwarnsystem" dar. Wenn dieses aber "prophylaktisch" entfernt wird, steht es für die Tiergesundheitsüberwachung als Signalgeber nicht mehr zur Verfügung. Bei derzeit bis zu 10% Aufzuchtverlusten im Flatdeckbereich besteht hier enormes tiergesundheitliches Handlungs- und Verbesserungspotenzial.

Aufgabe der vorliegenden Erfindung war es daher Substanzen bereitzustellen, die eine präventive und/oder therapeutische Behandlung von Schwanznekrosen, insbesondere bei Schweinen ermöglichen.

### Beschreibung der Erfindung

Die zuvor genannte Aufgabe wird entsprechend den Ansprüchen gelöst.

Ein Aspekt der vorliegenden Erfindung bezieht sich auf die Verwendung einer für Endotoxin bildenden Mikroorganismen, insbesondere für Bakterien der Spezies *Escherichia coli,* zytostatischen und/oder zytolytischen Substanz und/oder Verwendung eines Anti-Endotoxins zur Behandlung und/oder Prophylaxe von Schwanznekrosen bei Tieren, insbesondere von Schwanznekrosen bei Schweinen.

Ein weiterer Aspekt der vorliegenden Erfindung bezieht sich auf die Verwendung einer Substanz zur Behandlung und/oder Prophylaxe von Schwanznekrosen bei Tieren, insbesondere von Schwanznekrosen bei Schweinen, die derart gestaltet ist, dass diese als Antitoxin oder Antidot gegen Endotoxine von gramnegativen Bakterien wirkt.

Überaschenderweise konnte in Testversuchen festgestellt werden, dass Schwanznekrosen durch Endotoxine aus gramnegativen Bakterien ausgelöst werden.

Biochemisch handelt es sich bei Endotoxinen um Lipopolysaccharide (LPS), die sich aus speziesspezifisch unterschiedlichen Polysaccharidketten und einem relativ einheitlichen Lipid (Lipid A) zusammensetzen. Oft werden die Begriffe Endotoxin und Lipopolysaccharid als Synomym gebraucht. Endotoxine sind meist thermostabil. Ihre toxischen Eigenschaften sind durch das Lipid A bedingt seine Aktivität wird allerdings durch den Polysaccharid-Anteil modifiziert.

Endotoxine entfalten in Abhängigkeit verschiedener Faktoren, wie z.B. Eintrittspforte bzw. Wirkort und Konzentration auf der einen und Disposition der betroffenen Person auf der anderen Seite, unterschiedliche Wirkungen im Organismus. Freigesetzt z.B. im Rahmen einer mikrobiellen Vergiftung wirken sie systemisch. Sie können zu einem septischen Krankheitsbild mit plötzlich auftretenden hohen Fieber und Störungen der Blutgerinnung bis hin zum Multiorganversagen mit Schock führen. Es können drei klinische Symptomkomplexe in Abhängigkeit von der Expositionsart unterschieden werden:
- akute Effekte nach einmaliger Exposition
- Effekte nach wiederholter Exposition über einen längeren Zeitraum
- Effekte nach gleichzeitiger Exposition gegenüber Endotoxin und anderen Substanzen.

Es wird ein neues Arzneimittel zur Behandlung von Behandlung und/oder Prophylaxe von Schwanznekrosen bei Schweinen zur Verfügung gestellt, umfassend Anti-Shiga-Toxine die geeignet sind, Shiga-Toxine aus *E*. *coli* zu blockieren und zu neutralisieren.

In einer weiteren Ausführungsform der vorliegenden Erfindung werden die Tiere immunisiert, wodurch ein geimpftes Tier nach Erregerkontakt vor der Infektionskrankheit geschützt werden soll (Postexpositionsprophylaxe). Hierbei werden dem Tier unmittelbar nach Infektion mit beispielsweise einem Shiga-Toxin bindende Antikörper aus tierischer oder gentechnischer Produktion injiziert. Die einzusetzenden Antikörper, die gegen Epitope des Shiga-Toxins gerichtet sind, blockieren und neutralisieren dann das Shiga-Toxin. In bevorzugten Ausführungsformen der vorliegenden Erfindung werden humanisierte monoklonale Mausantikörper aus Hybridomazellen zur passiven Immunisierung eingesetzt.

Erfindungsgemäß können auch Substanzen, beispielsweise spezifische Antibiotika verwendet werden, die auf Shiga-Toxin-bildende *E. coli* zytostatisch und/oder zytolytisch wirken, um zu verhindern, dass sich die Shiga-Toxin-bildenden E. coli vor der Produktion des Schwanznekrose verursachenden Shiga-Toxins im Körper des Schweins, insbesondere im Körper eines Ferkels in den ersten Lebenswochen vermehren. Beispiele für geeignete Antibiotika sind Aminopenicilline, Ureidopenicilline, Cephalosporine, Carbapeneme, Chinolone, Cotrimoxazol. Aminoglycoside sowie Kombinationen der zuvor genannten (Gholamreza Darai, Michaela Handermann, Hans-Günther Sonntag und Lothar Zöller: Lexikon der Infektionskrankheiten des Menschen Erreger, Symptome, Diagnose, Therapie und Prophylaxe. 4. Auflage. Springer, 2012, ISBN 978-3-642-17158-1, S. 295, doi:10.1007/978-3-642-17158-1; Deutsche Gesellschaft für Infektiologie: EHEC und Antibiotikabehandlung, 1. Juni 2011). Besonders bevorzugt werden als zytostatisch und/oder zytolytisch Substanzen Amoxicillin, Neomycin und/oder Enrofloxacin verwendet. Vorzugsweise kommen die Antibiotika sehr früh zum Einsatz, da es sonst zu einer vermehrten Ausschüttung von Endotoxin kommen kann.

Die Applikation der erfindungsgemäßen Arzneimittel kann in oraler, parenteraler, inhalativer oder intranasaler Form erfolgen. Um die Therapiemöglichkeiten zu erweitern, können als orale Anwendungsformen Tabletten, Kapseln, Dragees, Granulate, Saft, Sirup, Suspensionen oder Lösungen eingesetzt werden. Hierbei ist die eingesetzte Arzneiform aus biologisch verwertbaren oder biologisch abbaubaren Stoffen ausgebildet, wobei die biologischen Materialien Proteine oder Proteide, Lipide oder Lipoide, Kohlenhydrate oder Polysaccharide, oder Mischungen aus mehreren solcher Materialien sind.

Um die Therapiemöglichkeiten zu erweitern, kann zusätzlich zum Anti-Shiga-Toxin bzw. zu Substanzen, die auf Shiga-Toxin-bildenden E. coli zytostatisch und/oder zytolytisch wirken, eine weitere Substanz, beispielsweise ein NSAID (z.B. Acetylsalicylsäure) in Kombination mit einem Antibiotikum eingesetzt werden. Weitere bevorzugte Ausführungformen sind die Kombination mit Zink-Oxid (wirkt hemmend auf *E. coli*) sowie die Kombination mit Toxinbindern als Futtermittel-Ergänzungsstoffe in Form von a) biotischen Toxinbindern (z.B. Grünmehle) und/oder b) abiotische Toxinbindern (insbesondere Tonminerale wie Betonite, Kaolinite, Zeolite). Bevorzugt werden diese weiteren Substanzen oral verabreicht.

Ferner bezieht sich die vorliegende Offenbarung auf ein Verfahren zur Herstellung einer pharmazeutischen Zusammensetzung zur Behandlung und/oder Prophylaxe von Schwanznekrosen bei Schweinen, das folgende Schritte aufweist:
(a) Bereitstellung einer Substanz, die für Shiga-Toxin-bildende *E. coli* zytostatisch und/oder zytolytisch ist und/oder eines Anti-Shiga-Toxins, und
(b) Formulierung dieser Substanz in einen pharmazeutisch akzeptablen Träger.

Erfindungsgemäß ergibt sich ein wichtiger und zugleich praktikabler Ansatzpunkt für eine geeignete Therapie von Schwanznekrosen bei Schweinen. Beispielsweise können Ferkel bereits in einer jungen Lebensphase (etwa am 4. Lebenstag) mit einem Anti-Shiga-Toxin (z.B. Stx2e) geimpft werden. Dieses Anti-Shiga-Toxin kann entweder als "Generikum" eigens hergestellt werden, oder es erfolgt eine Umwidmung bereits verfügbarer Impfstoffe auf das neue Anwendungsgebiet "Prophylaxe von Schwanznekrosen bei Schweinen".

Unter dem Begriff Shiga-Toxine werden im Zusammenhang mit der vorliegenden Offenbarung zytotoxische Proteine bezeichnet, die beispielsweise von dem Gram-negativen, stäbchenförmigen, fakultativ-anaeroben Bakterium *Shigella dysenteriae,* dem Erreger der Shigellosen oder Bakterienruhr gebildet werden. Ferner werden im Zusammenhang mit der vorliegenden Offenbarung unter dem Begriff Shiga-Toxine insbesondere zytotoxische Proteine bezeichnet, die von *Escherichia coli* (Enterohämorrhagische *Escherichia coli*) produziert werden (Vero-Toxine) und mit den von *Shigella dysenteriae* gebildeten Proteinen verwandt sind, beispielsweise Shiga Toxin 2e (Stx2e) (MacLeod et al. 1991).

Das gesamte Shiga-Toxin besitzt eine Molmasse von rund 70.000 Dalton; das Protein besteht aus zwei verschiedenen Untereinheiten, die über Disulfidbrücken miteinander verbunden sind. Die B-Untereinheit (7,6 kDa) ist fünffach vorhanden und sorgt für die Bindung an die Zelloberfläche und dafür, dass die A-Untereinheit (etwa 30 kDa) in das Zellinnere geschleust wird, wo diese die Eiweißsynthese durch Spaltung der 28s-rRNA der Ribosomen hemmt. Es besitzt einen ausgeprägten Neurotropismus.

Die Lektine Vero-Toxin 1 und Vero-Toxin 2 aus *Escherichia coli* werden aufgrund ihrer Ähnlichkeit zu Shiga-Toxin auch Shiga-like-toxin I/II (SLT I/II) oder Shiga-Toxin 1/2 (Stx1/Stx2) genannt. Sie beeinträchtigen die eukaryotische Proteinsynthese, indem sie die 60S-Untereinheit der Ribosomen auf katalytischem Weg inaktivieren. Die Elongation der Peptidkette wird dadurch beeinträchtigt, dass die Bindung von Aminoacyl-tRNA an die Ribosomen verhindert wird (Tesh et al., Infect Immun 1993;61:3392-402).

Von ihrer Struktur und Wirkungsweise her gehören Vero-Toxine und Shiga-Toxine zur gleichen Gruppe von Toxinen wie die Lektine Ricin und Abrin. Wie diese besitzen sie zwei Ketten bzw. Untereinheiten und das Bemerkenswerte daran ist, dass ihre jeweilige Untereinheit genau an der gleichen Stelle wie Ricin A eine Spaltung der N-glykosidischen Bindung von Adenin in der 28S-rRNA verursacht. Diese Toxine wirken als spezifische RNA-N-Glycosidasen, welche Ribosomen inaktivieren. In einer bevorzugten Ausführungsform der vorliegenden Erfindung werden Anti-Shigatoxine zur Vorbeugung und/oder Behandlung von Schwanznekrosen bei Schweinen verwendet.

Anti-Shigatoxine im Sinne der vorliegenden Erfindung sind rekombinante Fusionsproteine, die aus einem Fragment der Stx2eB-Untereinheit und der Glutathion-S-Transferase von *Shistosoma Japonicum* bestehen (Franke et al. 1995). Diese Fusionsproteine wurden bislang als potentielles Impfantigen zur Prophylaxe der Ödemkrankheit angesehen (Wieler L. H., Franke Sylvia, Rose M. und Karch H. Charakterisierung der Immunantwort bei der Ödemkrankheit des Schweines mit einer rekombinanten B-Untereinheit des Shiga-like-Toxins-Ile. Vortrag gehalten auf dem 21. DVG Kongress in Bad Nauheim (März 1995)).

Ferner wird von der vorliegenden Offenbarung die Verwendung von rekombinanten Fusionsproteinen umfasst, die ein subgenisches Stx2e-Fragment des Shiga-Toxins 2e in Fusion mit einem terminalen Tag, dessen Größe etwa der Größe des Fragments oder eines Bruchteils des Fragments entspricht aufweisen. Der terminale Tag ist eine markierte Endgruppe in der Aminosäurenfolge des Proteins. Vorzugsweise ist das subgenische Stx2e-Fragment eine B-Untereinheit (Stx2eB) des Shiga Toxins 2e. Diese Fusionsprotein und dessen Herstellung werden beispielsweise in der veröffentlichten internationalen Patentanmeldung WO 00/75345 beschrieben.

Weitere Anti-Shigatoxine im Sinne der vorliegenden Erfindung sind beispielsweise poly- oder monoklonale Antikörper, die gegen das Shiga-Toxin gerichtet sind. Kommerziell erhältlich sind unter anderem monoklonale IgG und IgG1 Mausantikörper aus Hybridomazellen gegen das *Escherichia Coli* Shigatoxin, inbesondere gegen die Shiga Toxin 2 Subunit A (SLT2A) oder die Subunit B. Ferner können Anti-Shiga Toxin (Stx) 2 humanisierte monoklonale Antikörper (siehe Yamagami et al., The Journal of Infectious Diseases, 2001 oder Nakao et al., Infect Immun 1999;67:5717-22, Volume 184) wie sie in zur Vorbeugung und/oder Behandlung von Schwanznekrosen bei Schweinen verwendet werden können. Die europäische Patentanmeldung EP 0817647 A1 beschreibt eine Vielzahl unterschiedlicher Shiga-Toxine sowie Anti-Toxine zur Behandlung von Vero-Toxin bildenden *E.coli,* die auch zur Behandlung von Schwanznekrosen gemäß der vorliegenden Offenbarung verwendet werden können.

Die vorliegende Erfindung betrifft somit Anti-Shiga Toxine zur therapeutischen oder/und prophylaktischen Behandlung von Schwanznekrosen bei Schweinen und die Verwendung dieser Substanz zur Herstellung eines Arzneimittels zur Behandlung und/oder Prophylaxe von Schwanznekrosen bei Schweinen.

Vorzugsweise werden die Anti-Shiga Toxine, insbesondere bei einer aktiven Immunisierung oder Schutzimpfung am 4. Lebenstag verabreicht. Zur Ausbildung einer belastbaren Immunität bedarf es etwa 2 Wochen, so dass beim Absetzen der Ferkel ab dem 21. Lebenstag dann eine belastbare Immunität besteht.

Ein weiterer Aspekt der vorliegenden Offenbarung bezieht sich auf Verfahren zur therapeutischen oder/und prophylaktischen Behandlung von Schweinen, die von Schwanznekrosen betroffen sind oder/und bei denen die Gefahr einer Schwanznekrose besteht, das folgende Schritte aufweist: (a) Bereitstellung einer Substanz, die für Shiga-Toxin-bildende E. coli zytostatisch und/oder zytolytisch ist, und (b) Einbringung der Substanz in das Lebewesen.

Besondere Ausführungsformen der vorliegenden Offenbarung beziehen sich daher auf die Verwendung einer für Shiga-Toxin bildende Bakterien der Spezies *Escherichia coli,* zytostatischen und/oder zytolytischen Substanz und/oder Verwendung eines Anti-Shigatoxins, insbesondere eines Anti-Shigatoxins gegen ein Shiga-Toxin aus *Escherichia coli,* zur Behandlung und/oder Prophylaxe von Schwanznekrosen bei Schweinen, wobei
a) die zytostatische und/oder zytolytsche Substanz ein gegen Shiga-Toxin bildende *Escherichia coli* wirkendes Antibiotikum sein kann,
b) das Anti-Shigatoxin ein rekombinantes Fusionsprotein umfassend ein Stx2e-Fragment des Shiga-Toxins 2e oder ein rekombinantes Fusionsprotein umfassend Fragmente der Stx2eB-Untereinheit des Shiga-Toxins 2e und der Glutathion-S-Transferase von *Shistosoma Japonicum.*
c) das Anti-Shigatoxin ein an das Shiga-Toxin bindender Antikörper sein kann, insbesondere ein monoklonaler IgG Antikörper gegen das *Escherichia Coli* Shiga-Toxin, inbesondere ein monoklonaler Antikörper gegen die Shiga-Toxin 2 Subunit A (SLT2A) und/oder die Subunit B.

Weitere Ausführungsformen der vorliegenden Erfindung beziehen sich auf Arzneimittel zur Behandlung und/oder Prophylaxe von Schwanznekrosen bei bei Schweinen umfassend eine für Shiga-Toxin bildende Bakterien der Spezies *Escherichia coli,* zytostatische und/oder zytotoxische Substanz, wobei
a) die Substanz ein Antibiotikum mit Wirkung gegen Shiga-Toxin bildende *Escherichia coli* sein kann,

Weitere Ausführungsformen der vorliegenden Erfindung beziehen sich auf Arzneimittel zur Behandlung und/oder Prophylaxe von Schwanznekrosen bei Schweinen umfassend ein Anti-Shigatoxin, insbesondere eines Anti-Shigatoxins gegen ein Shiga-Toxin aus *Escherichia coli,* wobei
a) das Anti-Shigatoxin ein rekombinantes Fusionsprotein ein Stx2e-Fragment des Shiga-Toxins 2e oder ein rekombinantes Fusionsprotein umfassend Fragmente der Stx2eB-Untereinheit des Shiga-Toxins 2e und der Glutathion-S-Transferase von *Shistosoma Japonicum.*
b) das Anti-Shigatoxin ein an das Shiga-Toxin bindender Antikörper sein kann, insbesondere ein monoklonaler IgG Antikörper gegen das *Escherichia Coli* Shigatoxin, inbesondere ein monoklonaler Antikörper gegen die Shiga Toxin 2 Subunit A (SLT2A) und/oder die Subunit B.

In konventionellen Haltungsformen kommt es beim Schwein häufig zu Schwanzentzündungen und Schwanzbeißen, oftmals auch in Verbindung mit Ohrrandentzündungen. Die zurzeit einzige gängige "Prophylaxe" ist das vorsorgliche Kürzen des Schwanzes beim Ferkel durch Amputation am vierten Lebenstag, was jedoch als "Routinemaßnahme" EU-rechtlich verboten ist.
Es konnte gezeigt werden, dass das Phänomen des Schwanzbeißens in zwei Formengruppen unterschieden werden muss:
1.) primärer Kannibalismus - zumeist als Frustrationsbeißen bei Beschäftigungsmangel oder bei Überbelegung der Ställe, auch als Folge von Fehlern im Haltungssystem selbst (Klima, Wasser); diese Form tritt meistens am Ende der Aufzuchtphase auf (4. - 6. Woche nach dem Absetzen), wenn die Tiere ein höheres Gewicht erreicht haben und die Belegdichte durch das Größenwachstum relativ zunimmt.
2.) sekundärer Kannibalismus - als Folge einer vorausgegangenen mit Juckreiz und Schmerzen verbundenen Nekrose des distalen Endes des Schwanzes. Ursache ist eine Entzündung des Blutgefäßendothels, die durch Verklebungen und Verlegungen der Blutendstrombahn zur Einschränkung der distalen Blutgefäßversorgung führt. Dies ist Gegenstand der nachstehenden Ausführungen. Diese Form tritt zumeist zu Beginn der Aufzuchtphase (2. Woche) auf.

Wie schon oben erwähnt, wurde gezeigt, dass die Hauptursachen für den Kannibalismus bei Schweinen (an den Schwanzspitzen und Ohrrändern), dem man in der Praxis durch "präventives" Kürzen des Schwanzes routinemäßig zu begegnen sucht, bereits bei sehr jungen Ferkeln ansetzen und prioritär in krankhaften Veränderungen im Magen-Darmbereich liegen. Den Schweinen mangelt es vor allem an einer ausreichenden Versorgung mit Strukturfutter (verbrauchsgerechtem, faserreichem Beschäftigungsmaterial), das die Darmflora stabilisiert. In Verbindung mit einem sehr frühen Absetzen vom Muttertier und der damit verbundenen unzureichenden Ausreifung des Magen-Darmtraktes, weiterhin begünstigt durch Fehler im Tränkemanagement (Wasserqualität und Tränketechnik) kommt es dann bei den Ferkeln zu Dysbiosen und Instabilitäten des Darmtraktes, deren Folgen bis in den Mastbereich reichen.

Im Ergebnis werden die Tiere also stoffwechselmäßig überfordert; das dysbiotische Darmmilieu begünstigt die Entstehung von Endotoxinen (insbesondere Shigatoxinen), die von dort ins Blut gelangen und entzündliche, subepitheliale Veränderungen in der peripheren Blutstrombahn verursachen. Die Folge ist eine Minderdurchblutung bis hin zur Ischämie mit Absterben des distal gelegenen Gewebes. Dies führt zu Entzündungen an Ohrrändern und Schwänzen (teilweise auch an den Flanken), wobei die damit verbundene Juckreizbildung zu gegenseitigem Beißen in jeglicher Ausprägungsform führt.

Daher ist ein weiterer Aspekt der vorliegenden Erfindung die Verwendung von Strukturkohlenhydraten zur Behandlung und/oder Prophylaxe von Schwanznekrosen bei Schweinen.

Nach ihrer Funktion im Organismus kann man die Kohlenhydrate in Strukturkohlenhydrate und Nicht-Strukturkohlenhydrate unterteilen, wobei Nicht-Strukturkohlenhydrate u. a. Rohrzucker (Saccharose) sowie das Polysaccharid Stärke sind. Diese Zucker dienen dem Energiegewinn oder sind Reservestoffe. Strukturkohlenhydrate sind insbesondere am Aufbau der pflanzlichen Zellwand beteiligt und stellen einen Großteil des Fasermaterials der Pflanzen dar wie Zellulose, Hemizellulose,

Strukturfutter im Sinne der vorliegenden Erfindung ist ein Futtermittel mit einem hohen Anteil an Strukturkohlenhydraten und umfasst beispielsweise Raufutter, d.h. Futtermittel mit einem relativ hohen Gehalt an strukturierter Rohfaser (z.B. Heu, Stroh, Luzernegrünmehl). Je nach Futtermittel und Art der Futterbergung ist ein unterschiedlich hoher Anteil der Rohfaser strukturwirksam. Dieser Anteil strukturwirksame Rohfaser (umgangssprachlich Strukturanteil) ist in Wiederkäuerrationen notwendig, um den Ruktus anzuregen, durch den das Futter aus dem Pansen wieder ins Maul gelangt und dort wiedergekaut wird. Durch den beim Wiederkauen gebildeten Speichel wird der pH-Wert im Pansen gepuffert. Rohfaserreiches Futter begünstigt die Essigsäureproduktion und damit die Milchfettbildung. Für andere Tierarten wie Schweine dient es neben der Anregung der Speichelproduktion (wichtig für den Säure-Basenhaushalt bei der Verdauung) auch zur Sättigung, ohne zu viel Energie bereitzustellen. Eine ausreichende Versorgung von Schweinen mit strukturwirksamer Rohfaser ist auch unmittelbar zur Stabilisierung der mikrobiellen Darmflora wichtig. So begünstigt ein Mangel an strukturwirksamer Rohfaser bei Schweinen eine Infektion mit Salmonellen, weil sich durch die damit verbundenen Veränderungen des Säure-Basen-Haushaltes im Darm die Lokalisationen für die jeweiligen Verdauungsleistungen vom Dickdarm in den distalen Dünndarm (Jejunum) verschieben,, was zu Dysbiosen führt. Eine ausreichende Versorgung von Schweinen mit strukturwirksamer Rohfaser ist daher eine wichtige Maßnahme in Schweinebeständen mit Salmonellenproblemen Daneben kann es auch (besonders Stroh) eine Funktion als Beschäftigungsmaterial übernehmen, was für die Mastschweine in den strukturarmen Ställen, aber auch für Pferde oder niedertragende Sauen wichtig ist.

Strukturfutter im Sinne der vorliegenden Erfindung umfasst ferner Grobfutter, d.h. nicht oder nur wenig zerkleinerte Pflanzen, die frisch oder getrocknet verfüttert werden können, wie Rüben, Maiskolben, die gesamte Maispflanze bzw. Stroh und Maisstroh. Grobfutter zeichnen sich durch eine hohe Strukturwirksamkeit aus. Bei Grobfutter-Zukauf (frisch) sind Silierverluste in Ansatz zu bringen (bei Gras 15 %, bei Mais 10 % jeweils auf die Trockenmasse bezogen).

Ferner können offenbarungsgemäß Endotoxinbinder zur Bindung von Endotoxinen, insbesondere von Endotoxinen aus *E.coli* zur Behandlung und/oder Prophylaxe von Schwanznekrosen bei Tieren, insbesondere von Schwanznekrosen bei Schweinen verwendet werden.

Beispiele für Endotoxinbinder sind die Lipopolysaccharid-bindenden Proteine LBP, BPI und rBPI21 (Beamer, L. J. 2003. Structure of human BPI (bactericidal/permeability-increasing protein) and implications for related proteins. Biochem. Soc. Trans. 31:791-794).

Zur Untersuchung der Ursache von Schwanznekrosen bei Schweinen, insbesondere bei Ferkeln wurde zunächst eine synoptische Gegenüberstellung mikrobiologischer Ursachen von Nekrosen der Akren bei Absatzferkeln durchgeführt. Tabelle 1 zeigt die Ergebnisse dieser Gegenüberstellung, wobei die Symbole folgende Bedeutung haben:
+ = Symptom tritt bei der jeweiligen Infektion auf
++ = Symptom ist typisch für die jeweilige Erkrankung,
(+) = Symptom kann auftreten, ist aber nicht charakteristisch für die jeweilige Erkrankung,
- = Symptom tritt nicht auf
Schwarz unterlegt = gramnegative Erreger
dunkel grau unterlegt = Mykoplasmen (pleomorph), hell grau unterlegt: grampositive Errger

**Tabelle 1**

| Befunderhebung in den Betrieben | E.coli (STEC) (Ödem-krankheit²) | Haemophilusparasuis (Glässersche Krankheit³) | Mykoplasma suis (Eperythro-Zoonose⁴) | Streptokokkus suis⁵ | Staphylococcus hyicus (Ferkelruß⁵) | Erysipelothrix rhuisopathiae (Rotlauf⁶) |
|---|---|---|---|---|---|---|
| Nekrose der Akren | ++ | + | ++ | + | + | + |
| Fieber | - | ++ | + | (+) | (+) | + |
| | | | | | | |
| gut entwickelte Ferkel | ++ | + | -/+ | -/+ | -/+ | -/+ |

Die Gegenüberstellung der verschiedenen Merkmale lässt vor allem im Formenkreis *E.coli* (STEC) eine hohe Übereinstimmung erkennen. Diese Ableitung stimmt auch mit den Beobachtungen aus 15 Feldbetrieben überein.

Hier wurden in Testreihen, bei sporadisch entnommenen Proben im Zusammenhang mit Schwanznekrosen bei Flatdeck-Ferkeln eine hohe Konzentration an *E. coli* und der Nachweis weit über der Norm liegender gramnegativer Lipopolysaccharide ("Endotoxine") geführt, was auf ein *E.coli*-Geschehen schließen lässt.

Die aufgefundenen überraschenden Ergebnisse werden dadurch gestützt, dass gegen eine primäre systemisch-infektiöse Ursache spricht, dass ansonsten die betroffenen Ferkel Krankheitserscheinungen im Sinne einer hochfieberhaften "Blutvergiftung" (= Bakteriämie) zeigen müssten. Dies gilt ebenso auch für Virus-induzierte Geschehen (= Virämie). Allerdings wurde festgestellt, dass sich die Situation unter Gabe von Antibiotika und NSAID's verbessert. Die Anmelderin kam somit zum Ergebnis, dass ein bakterieller Einfluss am Geschehen zumindest beteiligt sein muss.

Auch die Beobachtungen, dass es sich bei den analysierten Schwanznekrosen um klar abgegrenzte Prozesse handelt, sprechen gegen einen systemisch-infektiösen Verlauf, der in der Regel eher als disseminiertes Geschehen auftritt.

Wenn also eine primäre infektiöse Ursache als wenig wahrscheinlich erscheint, müssen folgerichtig alle bakteriellen Befunde (z.B. Strepto- und Staphylokokken) als sekundäres Geschehen gewertet werden, wie es im übrigen auch die klinischen Bilder und Krankheitsverläufe der Testversuche in den Betrieben zeigen.

Mykotoxine haben als Verursacher für Nekrosen vor allem beim Schwein eine große Bedeutung und dürften bei den beobachteten entzündlichen Prozessen in den Akren diese maßgeblich verursachen. Es wurde allerdings innerhalb der Testversuche festgestellt, dass dies als Ursache der Schwanznekrose ausgeschlossen werden kann, da die Wirksamkeit von Antibiotika nachgewiesen werden konnte.

Eine Infektion mit Mykoplasmen, so weit verbreitet sie in den Schweinebeständen auch vorkommen mögen, konnte aber anhand der Testreihen als primäre Ursache ausgeschlossen werden. Mykoplasmen leben überwiegend intrazellulär verursachen eine Schädigung zur Membran der roten Blutkörperchen, aber dieses Infektionsgeschehen verläuft eher multifokal. Im Verlauf der Infektion kommt es bedingt durch weitere Faktoren (Kälteagglutininen) zu Durchblutungsstörungen, quasi Mikrothrombosen, die im weiteren Verlauf zu Nekrosen in den Akren führen können.

Die Beobachtungen ergaben, dass eine Infektion mit Mykoplasmen nicht in dem Maße zu der jetzt festgestellten Trennschärfe von Demarkationslinien im Schwanz führt. Auch wäre mit einer Mykoplasmeninfektion nur schwerlich die unifokale, deutliche Demarkation der entzündlichen Stelle mit anschließendem reaktionslosen, trockenem "Abfallen" des veränderten Schwanzbereichs zu erklären.

Ferner lassen die Ergebnisse von Blutuntersuchungen betroffener Ferkel (Erhöhung des Anteils eosinophiler Granulozyten) darauf schließen, dass es sich um ein Toxin-vermitteltes Krankheitsgeschehen handelt. Dafür spricht auch, dass das Krankheitsgeschehen bei einer Steigerung der Wasseraufnahme, z.B: durch zusätzliche Gabe von Kochsalz (Natriumchlorid) über das Futter günstig beeinflusst werden konnte: die vermehrte Wasseraufnahme führt zu einer Verdünnung der Endotoxinkonzentration im Blut und mildert daher deren Auswirkungen klinisch erkennbar ab.

Alle erhobenen Befunde, Beobachtungen und Ableitungen sprechen insgesamt für ein toxisch induziertes Geschehen mit einer Schädigung an der Blutgefäßinnenwand, die ab einem bestimmten Gefäßlumen aufgrund von entzündungsbedingten Verklebungsprozessen an der Gefäßintima zu einem Gefäßverschluss führt mit der Folge, dass das distal gelegene Gewebe unterversorgt wird und daher anschließend abstirbt.

Diese vorstehenden Schlussfolgerungen, die hohe Prävalenz des Geschehens in konventionell geführten Betrieben, die betroffene Altersgruppe und auch die Feststellung, dass es sich bei den betroffenen Ferkeln stets um gut entwickelte, klinisch unauffällige Tiere handelt, führen zu dem Ergebnis, dass die Ursache im Umfeld des Formenkreises "Ödemkrankheit" (mit Bildung von Shigatoxin) zu finden ist. Gerade dieses Krankheitsbild ist bei der Zielgruppe sehr häufig: Bei Untersuchungen in hessischen Schweinebetrieben wurden Shigatoxin-Antikörper bei 53,2% der untersuchten Tiere nachgewiesen. Insgesamt konnten in 93 von 94 Betrieben serologisch positive Tiere identifiziert werden (Barth, S., Vallejo, G., Failing, K., Damriyasa, IM., Bauer, C., and Bauerfeind, R. 2003. Seroprevalence of Shigatoxin producing Escherichia coli in breeding sows in Hesse, Germany. Int. J. Med. Microbiol. 293: 139-140). Weltweit erkranken bis zu 20% der jährlich geborenen Ferkel mit klinischen Symptomen an der von STEC hervorgerufenen Ödemkrankheit (.http://www.iq-mitteldeutschland.de/iq/gewinner/iq-2012/cluster-biotechnologie-lifesciences.html).

In die gleiche Richtung (= bakterielle Grundbelastung mit gleichzeitiger Toxineinwirkung) weisen auch mehrere Berichte aus der Praxis, nach denen sich deutliche Besserungen nach Gabe von Antibiotika in Kombination mit NSAID's (z.B. Acetylsalicylsäure; zur Blutverflüssigung und Zellwandstabilisierung gegen Toxineinfluss) (Teich, K., Antibiotika einsparen Ja, aber wie?, Nutztierpraxis aktuell, Ausgabe 41, 2012)) zeigen. Ebenso konnte im Zuge der o.g. Verifizierungsstudie in acht Betrieben, die die Flatdeck-Ferkel im Zuge eines gravierenden Nekrose-/Beißgeschehens des Schwanzes mit einer Kombination von Acetylsalicylsäure und einen Antibiotkum behandelt haben, eine deutliche Besserung festgestellt werden. Eine besondere Wirkung scheint dabei die Acetylsalicylsäure zu spielen.

Neuesten wissenschaftlichen Untersuchungen zufolge zeigen feingewebliche Untersuchungen histopathologische Veränderungen an den kleinen Arterien und Arteriolen mit Schwellung des Endothels, hyaliner Degeneration und subendothelialer Fibrineinlagerung. In diesem Zusammenhang besonders erwähnenswert ist eine aktuelle Studie der Universität Wien bei 72 Ferkeln aus neun Betrieben im Alter von 5,5 bis 10 Wochen (zum Immunstatus: schutzgeimpft gegen Mycoplasma hyopneumoniae [s.o.]; ein Betrieb hat zusätzlich gegen PCV2 geimpft) (Weissenbacher-Lang, C., et al. 2012. Porcine ear necrosis syndrome: A preliminary investigation of putative infectious agents in piglets and mycotoxins in feed. The Veterinary Journal (2012)). Alle Ferkel zeigten die klinische Symptomatik des Ohrrandnekrosensyndroms im Anfangsstadium. In dieser Studie wiesen die kleinen und mittleren Blutgefäße vielfach Läsionen auf im Sinne einer Arteriosklerose, die für eine dystrophische Kalzifikation im Zusammenhang mit ausgedehnten subendothelialen Entzündungsprozessen (hier: der Blutgefäßintima) sprechen.

Vor allem die Fibrineinlagerung (die für den "verklebenden", Fibrin-absondernden Entzündungstyp beim Schwein typisch ist) begünstigt im weiteren Krankheitsverlauf die Verlegung des Blutgefäßlumens in der Endstrombahn mit der Folge, dass es distal des Gefäßverschlusses zur Ischämie mit Gewebenekrosen kommt.

Dass die krankheitsauslösenden Prozesse stets subendothelial, also außerhalb der eigentliche Zelle, ablaufen, ist ein weiteres wichtiges Ausschlussindiz gegen eine primär infektiöse Grunderkrankung, ausgelöst durch Viren oder Mykoplamen, weil diese Erreger streng intrazellulär leben. Subendotheliale Veränderungen sind dagegen eher typisch für toxisch induzierte Prozesse, wie sie etwa bei der Ödemkrankheit auftreten.

Alle erhobenen Befunde, Beobachtungen und Ableitungen sprechen insgesamt für ein durch Shiga-Toxine induziertes Krankheitsbild der Schwanznekrose bei Schweinen, das insbesondere mit einer Behandlung mit Anti-Shiga Toxinen verbessert werden kann und zur Verhinderung der Nekroseausbildung führt.

Soweit in Beschreibung auf Futtersubstanzen umfassend Strukturkohlenhydrate verwiesen wird, die auf endotoxinbildende Mikroorganismen hemmend wirken, konnte im Rahmen einer wissenschaftlichen Verifikationsstudie gezeigt werden, dass sowohl im Mastbereich als auch im Flatdeckbereich sich ein akutes Nekrose-/Beiß-Geschehen durch Gabe von Strukturfutter (hier: Maispflanzen bzw. Papiersäcke) spontan beruhigte. Dies entspricht auch den Beobachtungen im Mastbereich, bei dem ebenfalls die Struktur des Futters eine zentrale Rolle im Krankheitsgeschehen der Caudophagie zu spielen scheint.

Strukturfutter umfassend Strukturkohlenhydrate stabilisiert das Darmmilieu und hemmt somit das übermäßige Wachstum von endotoxinbildenden Bakterien wie *E.coli.* Genau in diese Richtung weist der Erfahrungsbericht eines Schweinehalters H. im Kreis Gütersloh. Dieser berichtete ganz konkret, dass er nach zusätzlicher Gabe von Luzernegrünmehl über das Futter bei Flatdeck-Schweinen das Problem von Schwanz- und Ohrrandnekrosen nunmehr gelöst habe. Darüber hinaus wurde die Wirksamkeit der zusätzlichen Gabe von Stroh bei Flatdeckferkeln in einem Betrieb mit ca. 1.500 Mastschweinen gezeigt.

Darüber hinaus konnte in einer gezielt initiierten Studie der Beweis erbracht werden, wie sehr eine ausreichende Versorgung der Flatdeck-Ferkel mit Strukturfutter Ohrrand- und Schwanznekrosen vorbeugen hilft. So wurden in der Studie 2 x 16 Flatdeck-Ferkel über die gesamte Aufzuchtperiode täglich mit eigens zugegebenen Strukturfutter (zwei ganze Maispflanzen) versorgt. Trotz unverändert bestehender Gesamtbestandsprobleme traten bei den entsprechend gefütterten Ferkel bis zum Ende der Flatdeck-Phase (Verbleib dort 2 Monate) keine Entzündungen oder Nekrosen auf, so dass damit die günstige Wirkung von Strukturfutter mit Strukturkohlenhydraten auf die Prophylaxe von Ohrrand- und Schwanznekrosen bewiesen ist.

Im weiteren zeitlichen Verlauf wurden zu den zentralen Kernaussagen (Darmgesundheit vs. Endotoxinbelastung mit Blutgefäßschädigung) weitere Beweise geführt, die im Folgenden dargestellt werden.

Beweise zur Aussage: das Geschehen wird durch Endotoxine (insbesondere Shigatoxine) getriggert:
Den kausalen Zusammenhang zwischen systemischer Endotoxinbelastung einerseits und der Nekrose des Schweineschwanzes andererseits belegen auch praktische Versuchsergebnisse, die zeigen, dass bereits bei jungen Saugferkeln bei der Sau Schwanznekrosen festgestellt wurden. So sind gerade bei säugenden Sauen hohe Endotoxinbelastungen bekannt (verursacht durch Futterbelastungen sowie durch den so genannten "MMA-Komplex", der mit bakterieller Belastung und hoher Endotoxinproduktion einhergeht). Aufgrund ihrer niedrig molekularen Struktur können die Endotoxine ungehindert die "Blut-Euter-Schranke" passieren und so auf das säugende Jungtier übergehen, was dann bei diesem die beschriebenen Gefäßschädigungen in der Endstrombahn (sichtbar an der Schwanzspitze) verursacht.

Ferner wurde in einem Betrieb ein spontan verendetes, gleichwohl gut entwickeltes Flatdeck-Ferkel (ca. 8. Lebenswoche) an der Tierärztlichen Hochschule Hannover untersucht. Dort wurden im Gehirn Streptokokken nachgewiesen. Bei gesunden Schweinen verhindert jedoch die "Bluthirnschranke" das Eindringen von Bakterien (Streptokokken) in das Gehirn; dieser Nachweis lässt somit zwingend den Schluss zu, dass bei diesem die Bluthirnschranke zerstört war, wie dies typischerweise bei Endotoxinen der Fall ist.

Wenn es sich um ein Endotoxin-vermitteltes Geschehen handelt, das von Darmbakterien ausgeht und über die Blutbahn verbreitet wird, muss es sich um einen systemischen, den gesamten Organismus betreffenden Prozess handeln. Dann müssten die entsprechenden Veränderungen nicht nur an den Ohrrändern und Schwanzspitzen (teilweise auch an den Flanken) erkennbar sein, sondern auch in anderen Organen im "Blutendstromgebiet", wie etwa an den Nieren. Um dies zu beweisen, wurden im Wege eines "Cross-Checks"von 12 Schlachtschweinen jeweils eine Niere entnommen und untersucht. Die Untersuchungsergebnisse zeigten deutlich ausgeprägte Gefäßentzündungen (interstitielle Nephritis), die typischerweise durch Toxine ausgelöst werden. Eine Virusinfektion (PCV-Infektion), die möglicherweise zu ähnlichen Veränderungen führen kann, wurde klinisch ausgeschlossen werden; zudem waren die Tiere gegen dieses Virus ohnehin schutzgeimpft.

Ferner konnte festgestellt werden, dass Acetylsalizysäure eine den Krankheitsverlauf beruhigende Wirkung aufweist.

Wie oben beschrieben hat die zusätzliche Gabe von NSAD (z.B. Acetylsalizylsäure) eine günstige Wirkung auf den Krankheitsverlauf der Schwanznekrose; Acetylsalizylsäure führt zu einer Verflüssigung des Blutes und hält somit bei einer vaskulären Vorschädigung mit ischämischer Tendenz eine bessere Durchblutung des Blutendstrombahngewebes aufrecht. Diese Erfahrungsberichte aus der Praxis und auch aus einem bestimmten Betrieb innerhalb einer Verifikationsstudie konnte jetzt zusätzlich in einem weiteren ausgewählten Betrieb in einer gezielten Studie bewiesen werden: Nachdem in diesem. Mastschweinebestand wiederholt und verbreitet Ohrrandnekrosen auftraten, wurden die Schweine in einer Bucht, die von diesem Geschehen besonders betroffen war, über einen Zeitraum von 4 Tagen mit Acetylsalizylsäure behandelt. Bei einer Nachkontrolle, die zwei Wochen nach der Behandlung erfolgte, konnte das fast vollständige Abheilen der Ohrrandnekrosen festgestellt werden, in den unbehandelten Nachbarbuchten war dagegen keine Besserung festzustellen.

Ferner konnte festgestellt werden, dass Kochsalz dem Krankheitsgeschehen vor beugt und eine metaphylaktische Wirkung hat eine den Krankheitsverlauf beruhigende Wirkung hat.

In der Beschreibung wird auf die günstige Beeinflussung des Krankheitsgeschehens durch zusätzliche Gabe von Kochsalz (Natriumchlorid) über das Futter verwiesen. Die zusätzliche Gabe von Natriumchlorid führt zu einer erhöhten Wasseraufnahme (Durst) und erleichtert die körpereigene Entgiftung (renale Exkretion).

Dies konnte jetzt in einem gezielten Fütterungsversuch ebenfalls konkret nachgewiesen werden: So wurden in einem Schweinehaltungsbetrieb, bei dem vor allem Ohrrandnekrosen als Bestandsproblem auftreten, bei 23 Flatdeck-Ferkeln von Beginn der Aufstallung bis zum Ende ein Futterrmittelergänzungspräparat beigemengt werden, dem als zusätzliche Komponente Natriumchlorid in einer Konzentration von 1% zugesetzt war. Der Gesamtanteil dieses Ergänzungsfuttermittels in der Ration betrug über die gesamte Vormastdauer 2%. Obwohl in diesem Betrieb auch während der Kontrollstudie ansonsten eine sehr hohe Prävalenz an Ohrrand- und Schwanznekrosen auftrat, blieben alle 23 so behandelten Ferkel zumindest bis zur 10. Lebenswoche (Beendigung der Studie) klinisch gesund.

Den Schwanzspitzennekrosen als primärer Wegbereiter für den Kannibalismus in Schweinebeständen kann wirksam vorgebeugt werden durch die gesamte Aufzucht- und Mastphase begleitende zusätzliche Gabe von
- Strukturkohlenhydraten enthaltend beispielsweise in Strukturfutter wie Heu, Stroh, Papier oder Maispflanzen);
- Toxinbindern zur Bindung von Endotoxinen ;
- Impfung gegen Endotoxine von Mikroorganismen wie *E.coli,* insbesondere gegen Shigatoxin

Bei allen "Versuchsgruppen", die unter kontrollierten Bedingungen mit Strukturfutter oder einem Toxinbinder versorgt wurden, lag die Ausfallquote bei 0 %. Dies verdient insofern besondere Erwähnung ,als in den untersuchten Betrieben die Verlustrate bei Flatdeck-Ferkeln üblicherweise ca. 3% beträgt, nach offiziellen Angaben bundesweit mit bis zu 10% sogar noch wesentlich höher.

### Testversuche:

### I. Genese des sekundären Kannibalismus (mit Nekrosebildung):

In Testversuchen konnte festgestellt werden, dass Schwanznekrosen durch ein Toxin aus Darmbakterien ausgelöst wird. Es handelt sich dabei um ein vom Bakterium *Escherichia coli* (E. coli) produziertes Toxin aus dem Formenkreis der Shiga-Toxine. Das Toxin zerstört den subendothelialen Bereich der Blutgefäße in den distal gelegenen Schwanzarealen und führt dort zu Entzündungen mit Verklebungen der Gefäßinnenauskleidung. Bei starker Ausprägung kann es zum Gefäßverschluss mit Ischämie kommen, was sich klinisch als Nekrose zeigt. Wie schon zuvor gezeigt wurden Shiga-Toxine bisher bei Schweinen bei Ödemkrankheit beschrieben, wobei es hier zu einer Zerstörung des subendothelialen Bereichs der Blutgefäße führt, klinisch sichtbar an Schwellungen insbesondere der Augelider und des Nasenrückens infolge des Flüssigkeitsaustritts aus den Blutgefäßen (deshalb der Name "Ödemkrankheit). Die Schwanznekrose ist daher dem erweiterten Formenkreis der "Ödemkrankheit" zuzuordnen.

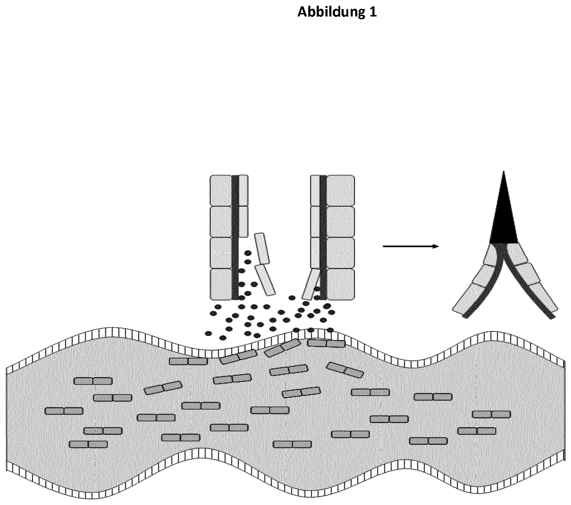

Wie in Abbildung 1 gezeigt, heften sich Toxin-bildende Bakterien an der inneren Darmschleimhaut des distalen Jejunums an. Dort produzieren sie das Toxin, das über die Blutstrombahn dann in die Peripherie gelangt (sichtbar an Ohrrändern und an der Schwanzspitze).

In den Testversuchen gelang es, den Toxin-bildenden Erreger durch ein gezielt wirkendes Antibiotikum an seiner Anhaftungsstelle selektiv abzutöten. Verwendet wurde das Antibiotikum Colistin in einer Konzentration von 125'000 IE/kg KGW/Tag (ca. 6 mg Colistinsulfat/kg KGW/Tag).. Dabei traten folgende Effekte auf:
a) Massives, plötzliches Ansteigen der Endotoxin-Konzentration im Blut aufgrund des bakteriellen Zerfalls unter dem Einfluss des Antibiotikums;
b) Entstehung einer großen "miliaren Wundfläche", da die Erreger unter dem Einfluss des Antibiotikums absterben. Dadurch entstehen an deren Anheftungsstellen miliare Wundflächen. Diese Anheftungsstellen sind zwar singulär gesehen sehr klein, verursachen jedoch in der Summe jedoch eine große Wundfläche, die sich aus miliaren Mikroläsionen zusammen setzt, weil die Erreger bei erkrankten Tieren massenhaft vorkommen

In den folgenden Abbildungen werden die Ergebnisse des "Toxin-Provokationstests" dargestellt, die bei Schweinen unter Feldbedingungen durchgeführt wurden (tägliche Blutkontrolle).

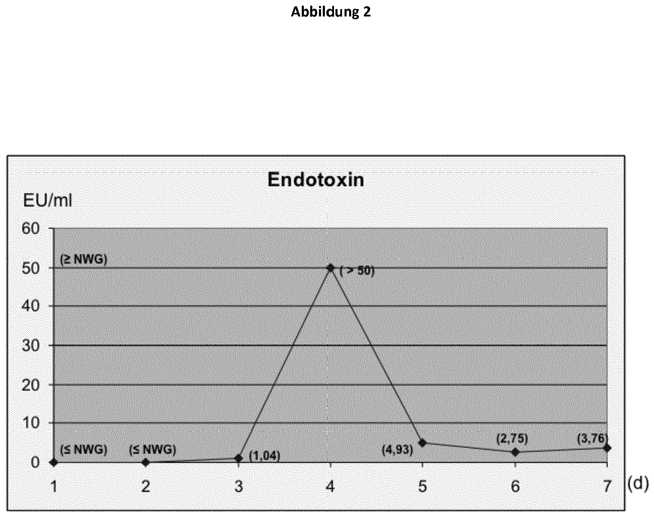

Abb. 2 zeigt, dass es ab dem dritten Tag nach Anwendung des Antibiotikums aufgrund des bakteriellen Zerfalls zu einem deutlichen Anstieg der Endotoxin-Fracht im Blut kommt.

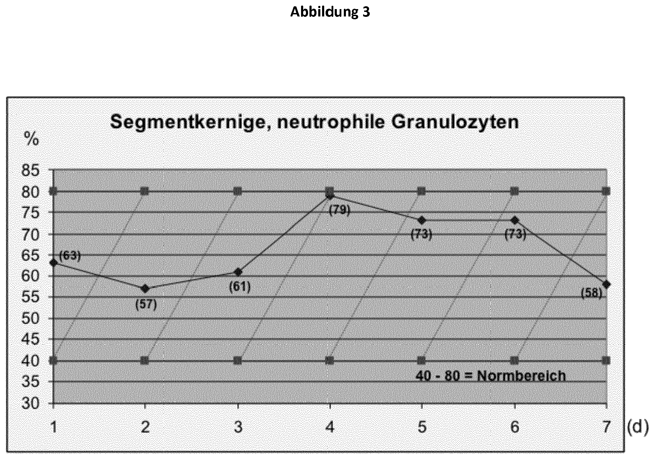

Die mit dem Zerfall der angehefteten Bakterien nunmehr frei gewordenen Anheftungspunkte liegen jetzt offen und sind miliare Wundstellen. Dadurch kommt es zu Entzündungen. Der Anstieg von segmentkernigen, neutrophilen Granulozyten (siehe Abb. 3) ist ein typisches Reaktionsmuster bei bakteriell bedingten Entzündungen (Eiterbildung); dies beweist die bakterielle Genese (E. coli). Der Anstieg der Entzündungszellen erfolgt parallel zum Zerfall der Bakterien ab dem 3. Tag p.appl.

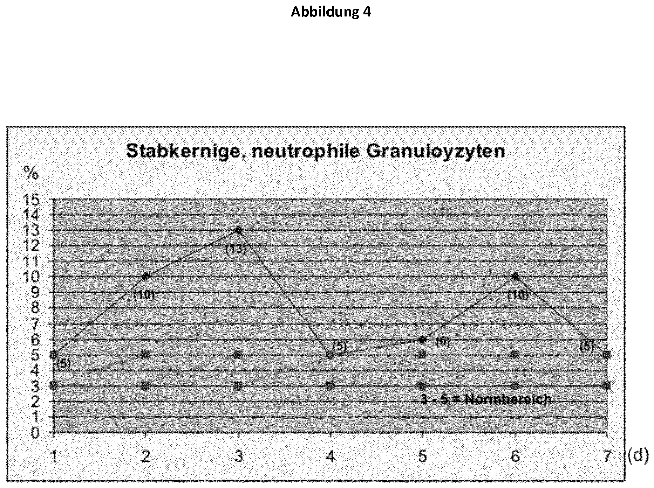

Abb. 4 zeigt, dass die Wundfläche entsprechend groß gewesen ist. So reichen die im Blut zirkulierenden (segmentkernige) Granulozyten nicht aus, um den Entzündungsprozess zu stabilisieren, und es werden daher zur Unterstützung der Entzündungsabwehr aus dem Knochenmark die dort schlummernden "Reservezellen" (stabkernige, neutrophile Granulozyten) mobilisiert. Wie sehr sich die im Blut zirkulierenden segmentkernigen Granulozyten durch die Entzündungsabwehr verbrauchen, wird an dem "Einbruch" der Menge im Blut frei zirkulierender segmentkerniger Granulozyten am 4. Tag deutlich. Nach der Phase der verbrauchsbedingten Erschöpfung setzt anschließend die Phase der "Stabilisierung" ein (6. Tag). Angesichts des Ausmaß des verbrauchsbedingten "Einbruchs" bei der Menge der im Blut zirkuleirenden segementkernigen Granulozyten muss die -dieses Entzündungsgeschehen auslösende- Wundfläche also im Ergebnis groß gewesen sein (eine große Vielzahl von angehefteten E.coli-Bakterien).

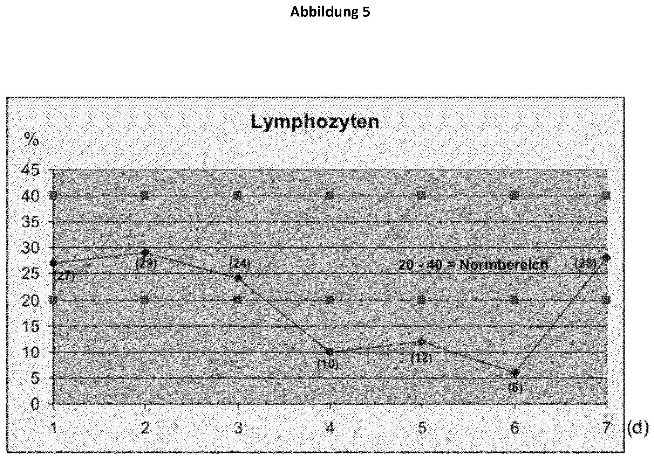

Lymphozyten sind für die körpereigene Immunabwehr wichtig, wirken jedoch nicht als "Sofortabwehr" sondern eher in einer Spätphase. Lymphozyten reagieren sehr empfindlich auf immunsuppresive Einflüsse (z.B. als Folge von Stress).. Der Einbruch der Lymphozytenkonzentration im Blut ab dem 4. Tag p.appl. lässt deutlich erkennen, dass das Tier nach der Behandlung Stress und (Bauch-)Schmerzen (infolge der Wundfläche) mit entsprechend immunsuppresiven Effekten gehabt hat (Abb.5). Erst bei beginnendem Heilungsprozess klingen diese Symptome ab, die immunsuppressive Wirkung lässt daraufhin nach und die lymphozytäre Abwehr kann sich wieder aufbauen.

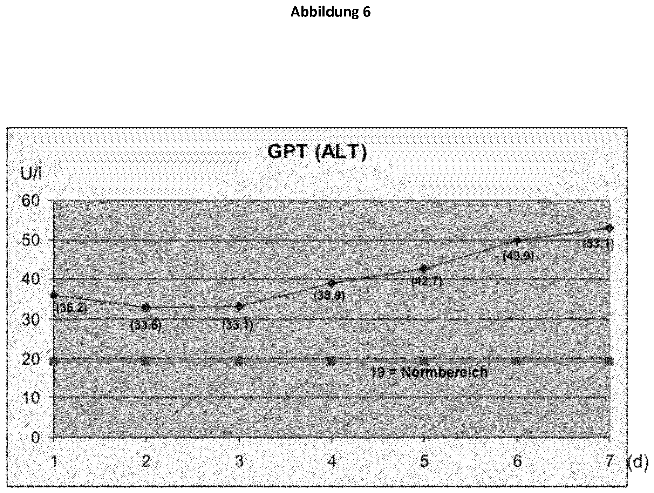

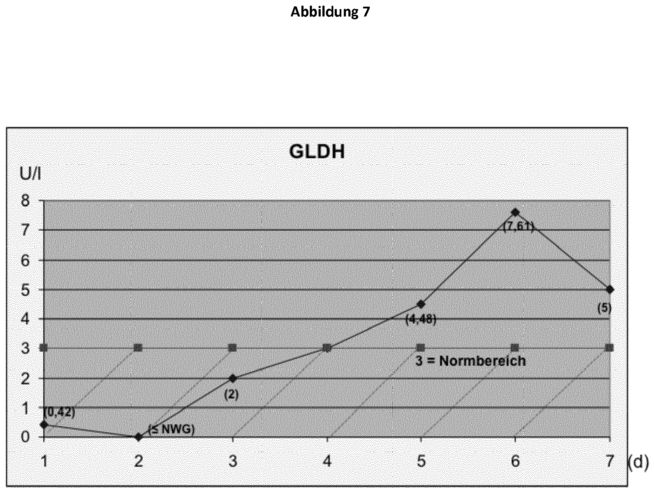

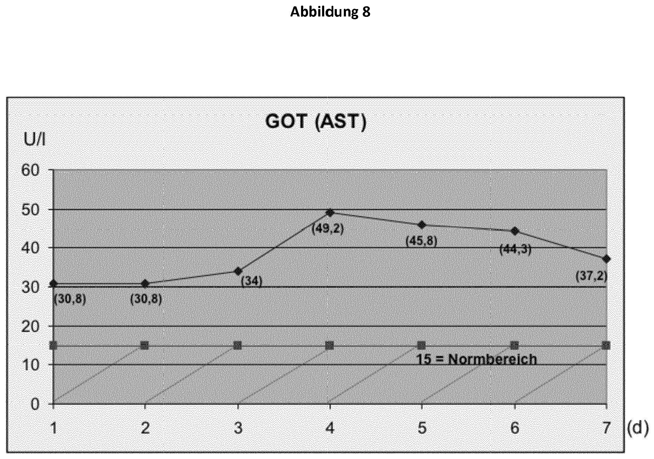

GLDH, GOT und GPT und sind Leberenzyme, die bei erhöhter Entgiftungsleistung (Toxin-Verstoffwechselung) im Blut ansteigen. Dass es sich hierbei um die freigesetzten Endotoxine handeln muss und andere Leberbelastungen sicher ausscheiden, beweist der in den Abb. 6,7 und 8 gezeigte spätere Abfall von GLDH und GOT wieder auf Normalwerte. Die Entgiftung wurde insoweit erfolgreich beendet.

Die Schwanznekrose konnte somit mittels Zugabe von Antibiotika bei den Versuchsschweinen geheilt werden.

### 2. Schwanznekrosen werden durch Toxin-vermittelte Entzündung des Gefäßendothels mit sekundären Verklebungen und Verlegungen des Gefäßlumens ausgelöst

Wie zuvor dargelegt, kommt es unter dem Einfluss von Endotoxinen zu einer Anschwellung des subendothelialen Bereiches in der Gefäßintima. Bei fortschreitendem Prozess kommt es zu Ablösungen und zu Entzündungsprozessen; diese führen zu Verklebungen und damit letztlich zur Verlegung des Gefäßlumens.

Dieser Pathomechanismus erklärt die gute Wirksamkeit von Acetylsalicylsäure (ASS) als Metaphylaxe oder auch als Therapeutikum bei bereits klinisch erkennbaren Schwanznekrosen. Denn zum Einen fördert ASS die Fließfähigkeit von Blut, was bei beginnenden Gefäßstenosen ("Verklebungen") für die Aufrechterhaltung der Durchblutung im Endstrombahngewebe wichtig ist und insofern in begrenztem Umfang eine Kompensation beginnender ischämischer Prozesse zu leisten vermag. Mindestens ebenso wichtig ist jedoch der zusätzliche Aspekt, dass unter dem Einfluss von ASS die subendotheliale Schwellung vermindert und so dem Entstehen von Intima-Entzündungen entgegen gewirkt wird. Dieser Wirkmechanismus erklärt, weshalb die Gabe von ASS allen anderen "Blutverdünnern" überlegen ist.

## Patentansprüche

1. Substanz zur Verwendung in der Behandlung und/oder Prophylaxe von Schwanznekrosen bei Schweinen, wobei die Substanz für Shiga-Toxin bildende Bakterien der Spezies *Escherichia coli* zytostatisch und/oder zytolytisch ist.

2. Substanz zur Verwendung nach Anspruch 1, **dadurch gekennzeichnet, dass** die Substanz ein gegen Shiga-Toxin bildende Bakterien der Spezies *Escherichia* coliwirkendes Antibiotikum ist.

3. Substanz zur Verwendung in der Behandlung und/oder Prophylaxe von Schwanznekrosen bei Schweinen, wobei die Substanz ein Anti-Shigatoxin ist, **dadurch gekennzeichnet, dass** das Anti-Shigatoxin ein rekombinantes Fusionsprotein umfassend Fragmente der Stx2eB-Untereinheit des Shiga-Toxins 2e und der Glutathion-S-Transferase von *Shistosoma Japonicum* oder ein an das Shiga-Toxin bindender Antikörper ist, insbesondere ein monoklonaler IgG Antikörper gegen das *Escherichia Coli* Shiga-Toxin, inbesondere ein monoklonaler Antikörper gegen die Shiga-Toxin 2 Subunit A (SLT2A) und/oder die Subunit B.

4. Strukturkohlenhydrate zur Verwendung in der Behandlung und/oder Prophylaxe von Schwanznekrosen bei Schweinen.

5. Arzneimittel zur Verwendung in der Behandlung und/oder Prophylaxe von Schwanznekrosen bei Schweinen umfassend eine für Shiga-Toxin bildende Bakterien der Spezies *Escherichia coli,* zytostatische und/oder zytotoxische Substanz.

6. Arzneimittel zur Verwendung in der Behandlung und/oder Prophylaxe von Schwanznekrosen bei Schweinen umfassend ein Anti-Shigatoxin, insbesondere eines Anti-Shigatoxins gegen ein Shiga-Toxin aus *Escherichia coli,* **dadurch gekennzeichnet, dass** das Anti-Shigatoxin ein rekombinantes Fusionsprotein umfassend Fragmente der Stx2eB-Untereinheit des Shiga-Toxins 2e und der Glutathion-S-Transferase von *Shistosoma Japonicum* oder ein an das Shiga-Toxin bindender Antikörper ist, insbesondere ein monoklonaler IgG Antikörper gegen das *Escherichia Coli* Shiga-Toxin, inbesondere ein monoklonaler Antikörper gegen die Shiga-Toxin 2 Subunit A (SLT2A) und/oder die Subunit B.

## Claims

1. Agent for the use in the treatment and/or prophylaxis of tail necrosis in pigs, wherein the agent is cytostatic and/or cytolytic for Shiga Toxin producing bacteria of the species *Escherichia coli.*

2. Agent for the use according to claim 1, **characterized in that** the agent is an antibiotic that is acting against a Shiga-Toxin producing bacteria of the species *Escherichia coli.*

3. Agent for the use in the treatment and/or prophylaxis of tail necrosis in pigs, wherein the agent is an anti-Shigatoxin, **characterized in that** the anti-Shigatoxin is a recombinant fusion protein comprising fragments of the Stx2eB-subunit of the Shiga-Toxin 2e and of the glutathion S-transferase of *Shistosoma Japonicum,* or **characterized in that** the anti-Shigatoxin is a Shiga-Toxin binding antibody, in particular a monoclonal IgG-antibody directed against the *Escherichia Coli* Shiga-Toxin, in particular a monoclonal antibody directed againt the Shiga-Toxin 2 Subunit A (SLT2A) and/or the Subunit B.

4. Structural carbohydrates for the use in the treatment and/or prophylaxis of tail necrosis in pigs.

5. Medicament for the use in the treatment and/or prophylaxis of tail necrosis in pigs comprising an agent that is cytostatic and/or cytolytic for Shiga Toxin producing bacteria of the species *Escherichia coli.*

6. Medicament for the use in the treatment and/or prophylaxis of tail necrosis in pigs comprising an anti-Shigatoxin, **characterized in that** the anti-Shigatoxin is a recombinant fusion protein comprising fragments of the Stx2eB-subunit of the Shiga-Toxin 2e and of the glutathion S-transferase of *Shistosoma Japonicum,* or **characterized in that** the anti-Shigatoxin is a Shiga-Toxin binding antibody, in particular a monoclonal IgG-antibody directed against the *Escherichia Coli* Shiga-Toxin, in particular a monoclonal antibody directed againt the Shiga-Toxin 2 Subunit A (SLT2A) and/or the Subunit B.

## Revendications

1. Substance pour une utilisation dans le traitement et/ou la prophylaxie des nécroses de la queue chez les porcs, dans laquelle la substance est cytostatique et/ou cytolytique pour les bactéries de l'espèce *Escherichia coli* produisant la toxine Shiga.

2. Substance pour une utilisation selon la revendication 1, **caractérisée en ce que** la substance est un antibiotique agissant contre les bactéries de l'espèce *Escherichia coli* produisant la toxine Shiga.

3. Substance pour une utilisation dans le traitement et/ou la prophylaxie des nécroses de la queue chez les porcs, dans laquelle la substance est une anti-toxine Shiga, **caractérisée en ce que** l'anti-toxine Shiga est une protéine de fusion recombinante comprenant des fragments de la sous-unité Stx2eB de la toxine Shiga 2e et de la glutathion-S-transférase de *Shistosoma Japonicum* ou un anticorps liant la toxine Shiga, en particulier un anticorps IgG monoclonal dirigé contre la toxine Shiga d'*Escherichia coli*, en particulier un anticorps monoclonal dirigé contre la sous-unité A de la toxine Shiga 2 (SLT2A) et/ou la sous-unité B.

4. Hydrates de carbone structurels pour une utilisation dans le traitement et/ou la prophylaxie des nécroses de la queue chez les porcs.

5. Médicament pour une utilisation dans le traitement et/ou la prophylaxie des nécroses de la queue chez les porcs comprenant une substance cytostatique et/ou cytolytique pour les bactéries de l'espèce *Escherichia coli* produisant la toxine Shiga.

6. Médicament pour une utilisation dans le traitement et/ou la prophylaxie des nécroses de la queue chez les porcs comprenant une anti-toxine Shiga, en particulier une anti-toxine Shiga dirigée contre la toxine Shiga provenant d'*Escherichia coli,* **caractérisée en ce que** l'anti-toxine Shiga est une protéine de fusion recombinante comprenant des fragments de la sous-unité Stx2eB de la toxine Shiga 2e et de la glutathion-S-transférase de *Shistosoma Japonicum* ou un anticorps liant la toxine Shiga, en particulier un anticorps IgG monoclonal dirigé contre la toxine Shiga d'*Escherichia coli,* en particulier un anticorps monoclonal dirigé contre la sous-unité A de la toxine Shiga 2 (SLT2A) et/ou la sous-unité B.
